# EUROPÄISCHE PATENTSCHRIFT

(11) **EP 1 392 248 B1**
(45) Veröffentlichungstag und Bekanntmachung des Hinweises auf die Patenterteilung: **08.08.2007**
(21) Anmeldenummer: 02730275.1
(22) Anmeldetag: 23.05.2002
(51) Int. Cl.: A61K 9/16, A61K 9/14, A61K 9/20

(54) **VERFAHREN ZUR HERSTELLUNG VON IMPLANTATEN MITTELS LÖSUNGSMITTELFREIER HERSTELLUNG EINES HOMOGENISATS**
IMPLANT MANUFACTURING PROCESS BY MEANS OF SOLVENT FREE PREPARATION OF A HOMOGENATE
PROCÉDÉ DE PRÉPARATION D'IMPLANTS MOYENNANT LA PRÉPARATION SANS SOLVANT D'UN HOMOGÉNÉISAT

(30) Priorität: 23.05.2001 DE 10125509; 27.02.2002 DE 10208382
(43) Veröffentlichungstag der Anmeldung: 03.03.2004
(73) Patentinhaber: HEXAL AG, 83607 Holzkirchen (DE)
(72) Erfinder: SCHÜTZ, Gregor, 83607 Holzkirchen (DE); FREUDENSPRUNG, Brigitte, 83607 Holzkirchen (DE)
(74) Vertreter: Boeters, Hans Dietrich
(86) Internationale Anmeldenummer: PCT/EP2002/005680
(87) Internationale Veröffentlichungsnummer: WO 2002/094226

(56) Entgegenhaltungen:
- EP-A- 0 778 304
- WO-A-00/35423
- WO-A-94/23698
- FR-A- 2 418 067
- US-A- 5 134 122

## Beschreibung

Die Erfindung betrifft ein Verfahren zur lösungsmittelfreien Homogenisierung von Polymeren und aktiven Bestandteilen durch Kaltmahlen mit Hilfe einer Kryomühle bei der ImplantatHerstellung.

Bei der Implantatherstellung wird gemäß dem Stand der Technik zuerst das Polymer in einem organischen Lösungsmittel (z.B. Dichlormethan) gelöst und anschließend mit einer wäßrigen oder organischen Lösung (z.B. methanolisch) des aktiven Bestandteils gemischt Nach ausgiebiger Durchmischung werden bei erhöhter Temperatur langsam die Lösungsmittel abgedampft. Zurück bleibt eine homogenisierte Mischung eines Polymers mit einem aktiven Bestandteil, die weiter zu einem Implantat verarbeitet wird.

Bei der Herstellung von Mikropartikeln wird der aktive Bestandteil oder seine wäßrige Lösung in einer organischen Lösung des Polymeren suspendiert, dispergiert bzw. emulgiert. Anschließend erfolgt bevorzugt eine Sprühtrocknung, d.h. die Wirkstoff-Polymer-Suspension wird in einem Luftstrom zerstäubt und getrocknet. Auf diese Weise erhält man eine homogenisierte Mischung von Polymer und aktivem Bestandteil in Form von Mikropartikeln.

Folgende Nachteile sind bei diesem herkömmlichen Homogenisierungsverfahren zu beobachten:
- Viele aktive Bestandteile sind wärmelabil, d.h. sie zersetzen sich bei höheren Temperaturen. Da bei der Evaporation der Lösungsmittel erhöhte Temperaturen von Nöten sind, besteht die Gefahr einer Reduzierung des Gehaltes an aktiven Bestandteilen und damit einhergehend die Zunahme von Verunreinigungen durch Abbauprodukte.
- Proteine können über die übliche "Solvent-Extraction-Evaporation"-Technik in der Regel nur schwerlich in das Polymer eingearbeitet werden, da durch die Verwendung von organischen Lösungsmitteln häufig die biologische Aktivität dieser Proteine stark absinkt.
- Da organische Lösungsmittel zur Homogenisierung verwendet und beim Evaporationsvorgang diese Lösungsmittel nicht vollständig entfernt werden können, treten vermehrt Verunreinigungen auf, und die Verträglichkeit ist in manchen Fällen weniger gut.
- Die Belastung der Umwelt durch Lösumgsmittelabfälle ist ganz erheblich.
- Diese Art der Homogenisierung ist sehr zeitintensiv und langwierig.

Aus FR 2 918 067 ist auch bekannt, bei Raumtemperatur in Polymere Zusätze einzuarbeiten, die zuvor kryotechnisch zerkleinert worden sind, Dieser Stand der Technik beschäftigt sich jedoch nicht mit Homogenisaten für Implantate und Mikropartikel. Stand der Technik, der hierfür relevant ist, läßt sich der folgenden Tabelle entnehmen.

### Herstellung von Implantaten bzw Mikropartikeln

| Prioritätsjahr | Herstellung in Gegenwart von organischen Lösungsmitteln | Herstellung in Gegenwart von Wasser | Herstellung durch Schmelzen |
|---|---|---|---|
| 1969 | DE 2 051 580 | | DE 2 051 580 |
| | Spalte 13 "A" und | | Spalte 14 "B"; |
| | Spalte 14 "C"; | | Spalte 12 Zeile14 |
| | Spalte 12 Zeile 14 | | |
| 1981 | EP 0 058 481 | | |
| | Seite 11 Zeile 44; | | |
| | Seite 14 Zeile 3 | | |
| 1996 | | WO 98/09 613 | |
| | | Seite 9 Zeile 12 | |
| 1997 | WO 99/20 253 | | |
| | Seite 21 Anspruch 1; | | |
| | Seite 24 Anspruch 22 | | |
| 1998 | WO 99/38 535 | WO 99/48 517 | |
| | Seite 24 Zeile 16, | Seite 4 Zeile 30, | |
| | Seite 25 Zeile 26, | Seite 8 Zeile 14, | |
| | Seite 11 Zeile 23 | Seite 7 Zeile 12 | |
| | | | |
| | WO 00/33 809 | WO 00/33 809 | |
| | Seite 5 Zeile 16, | loc. cit. | |
| | Seite 13 Zeile 5 | | |
| | | | |
| | WO 00/40 259 | | |
| | Seite 4 Zeilen 20/24; | | |
| | Seite 11 Anspruch 4 | | |
| 1999 | WO 00/66 087 | | |
| | Seite 28 Anspruch 1 | | |
| | | | |
| | WO 00/76 483 | | |
| | Seite 26 Anspruch 1 | | |

US 5, 134, 122 beschreibt die Lösungsmittelfreie Herstellung eines Homogenisats, welches zu Implantaten weiter unarbeitet werden kann. Die Herstellung wird bei einer Temperature von 5-25°C durchgeführt.

Die Aufgabe der Erfindung ist es, bei der Implantat-Herstellung ein Homogenisierungsverfahren zu entwickeln, das ohne organische oder wäßrige Lösungsmittel auskommt und frei von oben erwähnten Nachteilen ist.

Gemäß einer Ausführungsform wird die der Erfindung zugrundeliegende Aufgabe durch ein Verfahren zur lösungsmittelfreien Herstellung eines Homogenisats für Implantate gelöst, bei dem man
- ein oder mehrere Polymere und
- einen oder mehrere aktive Bestandteile
als Rohstoffe gemeinsam auf eine in der kryotechnik augewandte Temperature herabkühlt und gemeinsam unterhalb der Glasübergangstemperatur des (der) Polymeren homogenisiert und nach beendeter Homogenisierung auf Raumtemperatur bringt und das Homogenisat gewinnt und das erhaltene Homogenisat zu Implantat(en) weiterverarbeitet.

Die Rohstoffe können als Pulver oder Granulat eingesetzt werden.

Bei dem erfindungsgemäßen Verfahren kann man auf die Temperatur von Trockeneis oder eines Flüssiggases, insbesondere auf die Temperatur von flüssigem Stickstoff, herabkühlen.

Ferner kann man bei dem erfindungsgemäßen Verfahren auf die Arbeitstemperatur einer Kryomühle herabkühlen.

Beim erfindungsgemäßen Verfahren kann man die beiden Verfahrensschritte des Abkühlens und Homogenisierens mehrfach nacheinander durchführen.

Man kann mechanisch homogenisieren, insbesondere durch Mahlen. Insbesondere kann man mit Hilfe einer Kryomühle mahlen.

Ferner kann man das beim erfindungsgemäßen Verfahren erhaltene Homogenisat extrudieren und zu Implantat (en) portionieren. Hierfür kann beispielsweise auf den eingans zitierten Stand der Technik verwiesen werden, etwa auf WO 98/09 613.

Die erfindungsgemäße Aufgabe konnte also gelöst werden, indem man das/die für ein Implantat geeignete(n) Polymer(en) und mindestens einen aktiven Bestandteil lösungsmittelfrei in einer Kryomühle (Freezer Mill) homogenisiert. Beispiele für Kryomühlen lassen sich aus Obenauf et al. in SPEX CertiPrep Handbook of Sample Preparation and Handling, SPEX CertiPrep Inc., Metuchen (NJ), USA, 1999, entnehmen. Auf eine Temperaturerhöhung und das Anlegen eines Vakuums kann bei diesem Verfahren verzichtet werden. Das Homogenisat weist einen sehr hohen Homogenisierungsgrad auf, so daß die Oberfläche eines erfindungsgemäßen Implantats glatt und homogen ist.

Das erfindungsgemäße Homogenisierungsverfahren kann folgende Schritte umfassen:
a) Abwiegen des/der Polymers(en) und mindestens eines aktiven Bestandteils
b) Überführung der Rohstoffe in den Mahlzylinder der Kryomühle
c) Vorkühlen der Rohstoffmischung mit flüssigem Stickstoff
d) Homogenisierung, durch abwechselnde Intervalle von Mahlen und Kühlen
e) Auftauen des Mahlgutes auf Raumtemperatur.

Der Mahlzylinder der Kryomühle kann aus Polycarbonat oder Edelstahl sein. Üblicherweise befindet sich in dem Mahlzylinder frei beweglich ein Mahlkolben (Stößel), der abgerundete Enden besitzen kann. Dieser wird durch wechselnde elektromagnetische Felder im Mahlzylinder hin und her bewegt, so daß ein Mahl- bzw. Homogenisierungsprozeß in Gang gesetzt wird. Die Beladungsmenge der Kryomühle ist beliebig und kann dem Bedarf angepaßt werden.

Bei der Homogenisierung in der Kryomühle wechseln sich Intervalle von Kühlen und Mahlen ab. Die Intervalle können von zeitlich gleicher Dauer oder unterschiedlicher Dauer sein. Dies ist vom Polymer und aktiven Bestandteil abhängig. Der Mahlzylinder wird mit Hilfe von flüssigem Stickstoff gekühlt.

Die bei der erfindungsgemäßen Homogenisierung verwendeten Polymere sollten ein Gewichtsmittel des Molekulargewichts von 5000 bis 80 000, vorzugsweise 8000 bis 40000 und insbesondere 9000 bis 16000 besitzen und hinsichtlich ihrer Verträglichkeit für Implantate geeignet sein. Derartige Polymere sind z.B. Silikone, Poly(dioxanone), Poly(siloxane), Poly(glycolide), Ethylenvinylacetat-Copolymere, Poly(L-lactide), Poly(D,L-lactide), Poly(D,L-lactide-co-glycolide), Poly(L-lactide-co-trimethylen-carbonate) oder Poly-(L-lactide-co-D,L-lactide). Die bevorzugten Polymere sind Poly(L-lactide), Poly(D,L-lactide), Poly(D,L-lactide-co-glycolide) oder Poly(L-lactide-co-D,L-lactide).

Der bei der Homogenisierung eingebrachte aktive Bestandteil kann z.B. ein Vertreter aus der Wirkstoffgruppe der Anabolika, Androgene, Antiandrogene, Gestagene, Östrogene, Progestagene, Hypophysenvorderlappenhormone, Hypophysenhinterlappenhormone, Hypothalamushormone, Prostaglandine, immunostimulierenden Substanzen, Antiarrythmika, Analgetika, Antirheumatika, Antidiabetika, Osteoporosemittel, Lipidsenker, Zytostatika, Opiatantagonisten und/oder Antianämika sein.

Als aktiver Bestandteil können ein oder mehrere Vertreter aus der Gruppe der Anabolika verwendet werden, z. B. Nandrolon, Clostebol, Metenolon und/oder deren Derivate und/oder deren pharmazeutisch unbedenklichen Salze.

Als aktiver Bestandteil können ein oder mehrere Vertreter aus der Gruppe der Androgene und deren synthetische Analoga, z. B. Testosteron, Mesterolon und/oder deren Derivate und/oder deren pharmazeutisch unbedenklichen Salze verwendet werden.

Als aktiver Bestandteil können ein oder mehrere Vertreter aus der Gruppe der Antiandrogene verwendet werden, z. B. Cyproteron, Chlormadinon, Mestranol, Dienogest und/oder deren Derivate und/oder deren pharmazeutisch unbedenklichen Salze.

Als aktiver Bestandteil können ein oder mehrere Vertreter aus der Gruppe der Gestagene und deren synthetische Analoga verwendet werden, z. B. Medroxyprogesteron, Dydrogesteron, Norethisteron, Levonorgestrel, Lynestrenol, Hydroxyprogesteron, Medrogeston, Progesteron, Norgestimat, Gestoden und/oder deren Derivate und/oder deren pharmazeutisch unbedenklichen Salze.

Als aktiver Bestandteil können ein oder mehrere Vertreter aus der Gruppe der Östrogene und deren synthetische Analoga verwendet werden, z. B. Estradiol, Estriol, Ethinylestradiol, Prasteron und/oder deren Derivate und/oder deren pharmazeutisch unbedenklichen Salze.

Als aktiver Bestandteil können ein oder mehrere Vertreter aus der Gruppe der Progestagene und deren synthetische Analoga verwendet werden, z. B. Desogestrel, Etonogestrel und/oder deren Derivate und/oder deren pharmazeutisch unbedenklichen Salze.

Als aktiver Bestandteil können ein oder mehrere Vertreter aus der Gruppe der Hypophysenvorderlappenhormone und deren synthetische Analoga verwendet werden, z. B. Tetracosactid, Choriongonadotropin, Urofollitropin, Somatropin, Follitropin, Urogonadotropin, Menotropin und/oder deren Derivate und/oder deren pharmazeutisch unbedenklichen Salze.

Als aktiver Bestandteil können ein oder mehrere Vertreter aus der Gruppe der Hypophysenhinterlappennhormone und deren synthetische Analoga verwendet werden, z. B. Desmopressin, Terlipressin, Oxytocin, Argipressin und/oder deren Derivate und/oder deren pharmazeutisch unbedenklichen Salze.

Als aktiver Bestandteil können ein oder mehrere Vertreter aus der Gruppe der Hypothalamushormone (LH-Releasing Hormon) und deren synthetische Analoga verwendet werden, z. B. Cetrorelix, Corticorelin, Triptorelin, Leuprorelin, Gonadorelin, Ganirelix, Buserelin, Nafarelin, Goserelin, Somatostatin, Octreotid und/oder deren Derivate und/oder deren pharmazeutisch unbedenklichen Salze, insbesondere Leuprorelinacetat oder Goserelinacetat.

Als aktiver Bestandteil können ein oder mehrere Vertreter aus der Gruppe der Prostaglandine und deren synthetische Analoga verwendet werden, z. B. Alprostadil, Dinoproston, Dinoprost und/oder deren Derivate und/oder deren pharmazeutisch unbedenklichen Salze verwendet werden.

Als aktiver Bestandteil können ein oder mehrere Vertreter aus der Gruppe der immunostimulierenden Substanzen und deren synthetische Analoga verwendet werden, z. B. Interferone (α-, β-, γ-Typ), Interleukine (I, II, III, VI, XI etc), GM-CSF (Milodistim, Molgramostim, Sargramostim), M-CSF, G-CSF (Filgrastim, Lenograstim), Acemannan, Ancestim, Arbekacin, Forfenimex, Glycopin, Imiquimod, Imupedon, Leridistim, Methisoprinol, Murabutid, Pidotimod, Romurtid, Roquinimex, Thymalfasin, Thymocartin, Thymoctonan, Thymopentin, Ubenimex.

Als aktiver Bestandteil können ein oder mehrere Vertreter aus der Gruppe der Antiarrythmika verwendet werden, z. B. Adenosin, Orciprenalin, Aprindin, Amiodaron, Verapamil, Metoprolol, Esmolol, Chinidin, Sotalol, Digoxin, ß-Acetyldigoxin, Diltiazem, Lidocain, Mexiletin, Prajmalium, Phenytoin, Gallopamil, Propafenon, Detajmium, Flecainid, Atenolol, Oxprenolol und/oder deren Derivate und/oder deren pharmazeutisch unbedenklichen Salze.

Als aktiver Bestandteil können ein oder mehrere Vertreter aus der Gruppe der Analgetika/Antirheumatika verwendet werden, z. B. Morphin, Pethidin, Fentanyl, Pentazocin, Methadon, Levacetylmethadol, Buprenorphin, Nefopam, Tramadol, Dextropropoxyphen, Flupirtin, Meptazinol, Nalbuphin, Tilidin, Phenazon, Metamizol, Propyphenazon, Paracetamol, Phenylbutazon, Mofebutazon, Kebuzon, Acemetacin, Ibuprofen, Naproxen, Diclofenac, Ketoprofen, Indometacin, Lonazolac, Aceclofenac, Mefenaminsäure, Etofenamat, Tiaprofensäure, Azapropazon, Lomoxicam, Meloxicam, Piroxicam, Tenoxicam, Penicillamin, Chloroquin, Methotrexat, Auranofin, Natriumaurothiomalat, Oxaceprol, Leflunomid, Sulfasalazin, Celecoxib, Rofecoxib, Etanercept, Sitosterin, Hyarulonsäure, Flufenaminsäure, Felbinac, Campher, Propylnicotinat, Levomenthol, Benzylnicotinat und/oder deren Derivate und/oder deren pharmazeutisch unbedenklichen Salze.

Als aktiver Bestandteil können ein oder mehrere Vertreter aus der Gruppe der Antidiabetika und deren synthetische Analoga verwendet werden, z. B. Insulin, Miglitol, Metformin, Phenformin, Buformin, Glibenclamid, Tolbutamid, Glimepirid, Gliclazid, Glibornurid, Gliquidon, Glisoxepid, Pioglitazon, Rosiglitazon, Repaglinid und/oder deren Derivate und/oder deren pharmazeutisch unbedenklichen Salze.

Als aktiver Bestandteil können ein oder mehrere Vertreter aus der Gruppe Osteoporosemittel verwendet werden, z. B. Natriumrisedronat, Dinatriumpamidronat, Natriumibandronat, Natriumetidronat, Dinatriumclodronat, Natriumalendronat, Dinatriumtiludronat, Natriumfluorid, Natriumfluorophosphat und/oder deren Derivate, Dihydrotachysterol, Calcitonin und/oder deren Derivate und/oder deren pharmazeutisch unbedenklichen Salze.

Als aktiver Bestandteil können ein oder mehrere Vertreter aus der Gruppe der Lipidsenker verwendet werden, z. B. Fluvastatin, Simvastatin, Cerivastatin, Pravastatin, Lovastatin, Atorvastatin, Colestipol, Colestyramin, Xantinolnicotinat, Dextrothyroxin, Inositolnicotinat, Acipimox, Sitosterin, Benzafibrat, Fenofibrat, Clofibrat, Etofyllinclofibrat, Etofibrat, Gemfibrozil und/oder deren Derivate und/oder deren pharmazeutisch unbedenklichen Salze.

Als aktiver Bestandteil können ein oder mehrere Vertreter aus der Gruppe der Zytostatika verwendet werden, z. B. Aclarubicin, Nimustin, Doxorubicin, Cytarabin, Melphalan, Mitomycin, Bleomycin, Carmustin, Lomustin, Vinblastin, Estramustin, Cyclophosphamid, Daunarubicin, Etoposid, Epirubicin, Fludarabin, Fluorouracil, Gemcitabin, Ifosamid, Trofosfamid, Chlorambucil, Dactinomycin, Busulfan, Procarbazin, Mitoxantron, Bendamustin, Paclitaxel, Docetaxel, Temozolomid, Teniposid, Cisplatin, Camptothecin, Idarubicin, Vinca-Alkaloide und/oder deren Derivate und/oder deren pharmazeutisch unbedenklichen Salze.

Als aktiver Bestandteil können ein oder mehrere Vertreter aus der Gruppe der Opiat-Antagonisten verwendet werden, z. B. Naltrexon, Naloxon und/oder deren Derivate und/oder deren pharmazeutisch unbedenklichen Salze.

Als aktiver Bestandteil können ein oder mehrere Vertreter aus der Gruppe der Antianämika verwendet werden, z. B. Erythropoietin alfa und/oder Erythropoietin beta.

Als aktiver Bestandteil können auch Wachtumshormone, Endorphine, Tumornekrose-Faktor und deren Derivate verwendet werden.

Als aktiver Bestandteil kann auch Insulin verwendet werden.

Unter pharmazeutisch unbedenklichen Salzen der genannten aktiven Bestandteile werden unter anderem Säureadditionssalze verstanden. Diese kann man durch die Reaktion des in der freien Form vorliegenden aktiven Bestandteils mit pharmazeutisch unbedenklichen Säuren erhalten. Die pharmazeutisch unbedenklichen Säuren können anorganische Säuren (z.B. Salzsäure, Bromwasserstoffsäure, Schwefelsäure, Salpetersäure, Phosphorsäure) oder organische Säuren sein (z.B. Essig-, Propion-, Hydroxyessig-, Milch-, Brenztrauben-, Oxal-, Malein-, Malon-, Bernstein-, Fumar-, Äpfel-, Wein-, Citronen-, Methansulfon-, Ethansulfon-, Benzolsulfon-, p-Toluolsulfon-, Cyclohexansulfamin-, Salicyl-, p-Aminosalicyl- und Pamoasäure).

Ebenso als Säureadditionssalze werden Solvate mit dem Wirkstoff bezeichnet. Derartige Solvate sind z.B. Hydrate oder Alkoholate.

Als mögliche pharmazeutisch unbedenkliche Salze der genannten aktiven Bestandteile kommen ebenso Alkalimetall- und/oder Erdalkalimetallsalze sowie das Ammoniumsalz in Frage, wie z.B. das Kalium-, Natrium-, Lithium-, Calcium-, Magnesium- oder Ammoniumsalz.

Das Verhältnis von Polymer(en) zu aktivem Bestandteil wird derart gewählt, daß der Gehalt an aktivem(n) Bestandteil(en) 5 bis 50 Gew.% bezogen auf das Homogenisatgewicht beträgt.

Die Erfindung wird durch nachstehende Beispiele näher erläutert.

### Beispiel 1:

Es werden 1,5 g Leuprorelinacetat und 4,5 g Poly-(D,L-lactid) (R202H) abgewogen und in den Polycarbonat-Mahlzylinder der Kryomühle (SPEX CertiPrep 6800 Freezer/ Mill) gegeben. Die Mischung wird 15 Minuten mit flüssigem Stickstoff im Mahlzylinder vorgekühlt. In diesem Mahlzylinder befindet sich zudem ein Mahlkolben mit stark abgerundeten Ecken. Zuerst wird 2 Minuten gekühlt, dann 2 Minuten mit 10 HZ gemahlen. Dieses Intervall wird wiederholt. Dann wird der Mahlbehälter verschlossen aufgetaut. Anschließend wird extrudiert, geschnitten und gewogen; die Implantate werden verpackt und in Fertigspritzen eingeführt.

### Beispiel 2:

Es werden 1,5 g Leuprorelinacetat und 4,5 g Poly-(D,L-lactid-co-glycolid) abgewogen und in den Polycarbonat-Mahlzylinder der Kryomühle (SPEX CertiPrep 6800 Freezer/ Mill) gegeben. Die Mischung wird 15 Minuten mit flüssigem Stickstoff im Mahlzylinder vorgekühlt. In diesem Mahlzylinder befindet sich zudem ein Mahlkolben mit stark abgerundeten Ecken. Zuerst wird 2 Minuten gekühlt, dann 2 Minuten mit 12 HZ gemahlen. Dieses Intervall wird wiederholt. Dann wird der Mahlbehälter verschlossen aufgetaut. Anschließend wird extrudiert.

### Beispiel 3:

Es werden 1,5 g Leuprorelinacetat und 4,5 g Poly-(D,L-lactid-co-glycolid) abgewogen und in den Polycarbonat-Mahlzylinder der Kryomühle (SPEX CertiPrep 6800 Freezer/ Mill) gegeben. Die Mischung wird 15 Minuten mit flüssigem Stickstoff im Mahlzylinder vorgekühlt. In diesem Mahlzylinder befindet sich zudem ein Mahlkolben mit stark abgerundeten Ecken. Zuerst wird 2 Minuten gekühlt, dann 2 Minuten mit 8 HZ gemahlen. Dieses Intervall wird wiederholt. Dann wird der Mahlbehälter verschlossen aufgetaut. Anschließend wird extrudiert.

### Beispiel 4:

Es werden 1,5 g Leuprorelinacetat und 4,5 g Poly-(D,L-lactid) abgewogen und in den Polycarbonat-Mahlzylinder der Kryomühle (SPEX CertiPrep 6800 Freezer/ Mill) gegeben. Die Mischung wird 15 Minuten mit flüssigem Stickstoff im Mahlzylinder vorgekühlt. In diesem Mahlzylinder befindet sich zudem ein Mahlkolben mit stark abgerundeten Ecken. Zuerst wird 2 Minuten gekühlt, dann 2 Minuten mit 12 HZ gemahlen. Dieses Intervall wird wiederholt. Dann wird der Mahlbehälter verschlossen aufgetaut. Anschließend wird extrudiert.

### Beispiel 5:

Es werden 1,5 g Leuprorelinacetat und 4,5 g Poly-(D,L-lactid) abgewogen und in den Polycarbonat-Mahlzylinder der Kryomühle (SPEX CertiPrep 6800 Freezer/ Mill) gegeben. Die Mischung wird 15 Minuten mit flüssigem Stickstoff im Mahlzylinder vorgekühlt. In diesem Mahlzylinder befindet sich zudem ein Mahlkolben mit stark abgerundeten Ecken. Zuerst wird 2 Minuten gekühlt, dann 2 Minuten mit 10 HZ gemahlen. Dieses Intervall wird viermal wiederholt. Dann wird der Mahlbehälter verschlossen aufgetaut. Anschließend wird extrudiert.

### Beispiel 6:

Es werden 1,5 g Goserelinacetat und 4,5 g Poly-(D,L-lactid) abgewogen und in den Polycarbonat-Mahlzylinder der Kryomühle (SPEX CertiPrep 6800 Freezer/ Mill) gegeben. Die Mischung wird 15 Minuten mit flüssigem Stickstoff im Mahlzylinder vorgekühlt. In diesem Mahlzylinder befindet sich zudem ein Mahlkolben mit stark abgerundeten Ecken. Zuerst wird 2 Minuten gekühlt, dann 2 Minuten mit 10 HZ gemahlen. Dieses Intervall wird fünfmal wiederholt. Dann wird der Mahlbehälter verschlossen aufgetaut. Anschließend wird extrudiert.

### Beispiel 7:

Es werden 3,0 g Leuprorelinacetat und 9,0 g Poly-(D,L-lactid) abgewogen und in den Polycarbonat-Mahlzylinder der Kryomühle (SPEX CertiPrep 6800 Freezer/ Mill) gegeben. Die Mischung wird 15 Minuten mit flüssigem Stickstoff im Mahlzylinder vorgekühlt. In diesem Mahlzylinder befindet sich zudem ein Mahlkolben mit stark abgerundeten Ecken. Zuerst wird 2 Minuten gekühlt, dann 2 Minuten mit 10 HZ gemahlen. Dieses Intervall wird dreimal wiederholt. Dann wird der Mahlbehälter verschlossen aufgetaut. Anschließend wird extrudiert.

### Beispiel 8:

Es werden 6,0 g Leuprorelinacetat und 18,0 g Poly-(D,L-lactid) abgewogen und in den Polycarbonat-Mahlzylinder der Kryomühle (SPEX CertiPrep 6800 Freezer/ Mill) gegeben. Die Mischung wird 15 Minuten mit flüssigem Stickstoff im Mahlzylinder vorgekühlt. In diesem Mahlzylinder befindet sich zudem ein Mahlkolben mit stark abgerundeten Ecken. Zuerst wird 2 Minuten gekühlt, dann 2 Minuten mit 10 HZ gemahlen. Dieses Intervall wird dreimal wiederholt. Dann wird der Mahlbehälter verschlossen aufgetaut. Anschließend wird extrudiert.

## Patentansprüche

1. Verfahren zur Herstellung von Implantat (en), bei dem man lösungsmittelfrei ein Homogenisat herstellt, indem man
- ein oder mehrere Polymere und
- einen oder mehrere aktive Bestandteile
als Rohstoffe gemeinsam auf eine in der Kryotechnik angewandte Temperatur herabkühlt und gemeinsam unterhalb der Glasübergangstemperatur des (der)-Polymeren homogenisiert und nach beendeter Homogenisierung auf Raumtemperatur bringt und das Homogenisat gewinnt und das erhaltene Homogenisat zu Implantat(en) weiterverarbeitet.

2. Verfahren nach Anspruch 1, **dadurch gekennzeichnet, dass** man die Rohstoffe als Pulver oder Granulat einsetzt.

3. Verfahren nach einem der vorhergehenden Ansprüche, **dadurch gekennzeichnet, dass** man auf die Temperatur von Trockeneis oder eines Flüssiggases, insbesondere auf die Temperatur von flüssigem Stickstoff herabkühlt.

4. Verfahren nach einem der vorhergehenden Ansprüche, **dadurch gekennzeichnet, dass** man auf die Arbeitstemperatur einer Kryomühle herabkühlt.

5. Verfahren nach einem der vorhergehenden Ansprüche, **dadurch gekennzeichnet, dass** man die beiden Verfahrensschritte des Abkühlens und Homogenisierens mehrfach nacheinander durchführt.

6. Verfahren nach einem der vorhergehenden Ansprüche, **dadurch gekennzeichnet, dass** man mechanisch homogenisiert, insbesondere durch Mahlen.

7. Verfahren nach Anspruch 6, **dadurch gekennzeichnet , dass** man mit Hilfe einer Kryomühle mahlt.

8. Verfahren nach einem der vorhergehenden Ansprüche, **dadurch gekennzeichnet, dass** man das erhaltene Homogenisat extrudiert und zu Implantate(en) portioniert.

## Claims

1. Method of producing implant(s) in which a homogenisate is prepared without solvent, wherein
- one or more polymers and
- one or more active constituents,
as raw materials, are cooled together to a temperature applied in cryogenic technology and are homogenised together below the glass transition temperature of the polymer(s) and, when homogenisation is complete, brought to room temperature and the homogenisate is obtained, and the resulting homogenisate is further processed to form implant(s).

2. Method according to claim 1, **characterised in that** the raw materials are used in the form of powders or granules.

3. Method according to one of the preceding claims, **characterised in that** cooling is carried out to the temperature of dry ice or of a liquid gas, especially to the temperature of liquid nitrogen.

4. Method according to one of the preceding claims, **characterised in that** cooling is carried out to the operating temperature of a cryogenic mill.

5. Method according to one of the preceding claims, **characterised in that** the two process steps of cooling and homogenisation are carried out repeatedly in succession.

6. Method according to any one of the preceding claims **characterised in that** homogenisation is carried out mechanically, especially by grinding.

7. Method according to claim 6, **characterised in that** grinding is carried out with the aid of a cryogenic mill.

8. Method according to any one of the preceding claims, **characterised in that** the homogenisate obtained is extruded, and is divided into portions to form implant(s).

## Revendications

1. Procédé pour la fabrication d'implants moyennant la fabrication sans solvant d'un homogénéisat, dans lequel on refroidi en même temps :
- un ou plusieurs polymères et
- un ou plusieurs composants actifs
en tant que matière première, à une température utilisée en cryotechnique et on homogénéise ensemble en dessous de la température de transition vitreuse du/des polymère(s) puis on amène à température ambiante après homogénéisation et on obtient l'homogénéisat et on transforme l'homogénéisat obtenu en implant(s).

2. Procédé selon la revendication 1, **caractérisé en ce que** l'on exploite les matières premiers sous forme de poudre ou de granulés.

3. Procédé selon l'une des revendications précédentes, **caractérisé en ce que** l'on refroidi à la température de glace carbonique ou d'un gaz liquide, en particulier à la température d'azote liquide.

4. Procédé selon l'une des revendications précédentes, **caractérisé en ce que** l'on refroidi à la température d'un cryopulvérisateur.

5. Procédé selon l'une des revendications précédentes, **caractérisé en ce que** l'on reproduit plusieurs fois à la suite les deux procédés de refroidissement et d'homogénéisation.

6. Procédé selon l'une des revendications précédentes, **caractérisé en ce que** l'on homogénéise mécaniquement, en particulier en pulvérisant.

7. Procédé selon la revendication 6, **caractérisé en ce que** l'on pulvérise à l'aide d'un cryopulvérisateur.

8. Procédé selon l'une des revendications précédentes, **caractérisé en ce que** l'on extrude l'homogénéisat obtenu et le portionne en implant(s).
